# EUROPEAN PATENT SPECIFICATION

(11) **EP 3 419 720 B1**
(45) Date of publication and mention of the grant of the patent: **19.08.2020**
(21) Application number: 17757449.8
(22) Date of filing: 24.04.2017
(51) Int. Cl.: A61N 5/06, A61F 7/00

(54) **LED THERAPY BED**
BETT FÜR LED-THERAPIE
LIT DE SOINS À DIODES ÉLECTROLUMINESCENTES

(30) Priority: 19.12.2016 US 201515383376
(43) Date of publication of application: 02.01.2019
(73) Proprietor: Marchese, Stephen James, Irvine, California 92606 (US); Marchese, Chasen James, Irvine, California 92606 (US)
(72) Inventor: Marchese, Stephen James, Irvine, California 92606 (US); Marchese, Chasen James, Irvine, California 92606 (US)
(74) Representative: Browne, Robin Forsythe
(86) International application number: PCT/US2017/029150
(87) International publication number: WO 2017/147622

(56) References cited:
- US-A1- 2006 253 175
- US-A1- 2014 207 215
- US-A1- 2015 265 846
- US-B1- 6 494 901
- US-B2- 8 425 577

## Description

### BACKGROUND OF THE INVENTION

### Field of the invention

This invention relates to improvements in a light emitting (LED) therapy bed. More particularly, the LED therapy bed provides a dermal therapy bed that can provide health benefits to a person by elevating and maintaining the therapy temperature of a person on the bed as a source of bio-stimulative non-coherent, non-monochromatic light.

This invention further relates to improvements in medical devices for topical photodynamic therapy (POT) treatment to patients, and particularly to a rigid surface (circuit board) containing LEDs as a source of bio-stimulative, non-coherent, non-monochromatic light, which are placed in contact or in close proximity to the patient's skin or tissue, and a method for making that apparatus.

### Description of Related Art

Non-monochromatic light at defined wavelengths has produced beneficial bio-stimulative effects and has been known to trigger specific biological functions, such as increased rate of metabolism, photo-repair and cell division. Stimulation occurred, however, with light emitted in specific wavelengths.

While the exact mechanism by which the beneficial bio-stimulative effects have been achieved is not precisely known, several theories have been put forth. It has been suggested that non-monochromatic light emitted in the range of about 415 to about 940nm penetrates body tissue and is absorbed, reflected and scattered to excite molecules within cells and tissue to thereby accelerate repair and regeneration. It is known however that light in the range of about 415nm to about 465nm has a bactericidal effect, thereby relieving the appearance of bacteria induced acne.

A further theory suggests that different cells had different photoreceptors, which respond to only particular wavelengths of light. This theory supported the phenomenon that the application of only certain wavelengths of light result in bio-stimulative effects and the resulting stimulation of the dermis and an increase of collagen and elastin production. With respect to similar but non-LED technology, light therapy has utilized lasers with relatively low power and bio-stimulative treatment utilizing lasers has been referred to as "soft" laser therapy. In such applications, low level laser energy radiation has been successfully employed to stimulate wound healing and treatment of musculoskeletal disorders and skin ulcers.

It has been previously theorized that the properties of laser radiation, which resulted in the beneficial bio-stimulative effects of soft laser therapy, were the monochromaticity and coherence of laser radiation.

In a prior invention, the applicant noted that if bio-stimulative light effects were compounded by combining into one device four different wavelengths of light, each with known benefits, that the effects could be greater than if each wavelength was applied separately, and that close proximity of the LEDs to the user promoted uniform coverage of the target area, in order to receive all wavelengths simultaneously, and more effective penetration of light.

Therapy beds have taken a variety of shapes and functions over the years. Some early therapy beds claimed to provide the health benefits of the sun, while later versions provided mostly tanning effects to the skin of the user.

A number of patents and/or publications relate to these issues. Exemplary examples of patents and/or publication that try to address this /these issues(s) are identified and discussed below.

U.S. Patent Number 6,896,693 discloses a "Photo-Therapy Device". The photo-therapy device of the patent operates up to several feet from the user.

U.S. Patent Number 8,425,577 discloses a "LED Phototherapy Apparatus". This patent uses red and near infra-red light to provide phototherapy. This patent also uses an acrylic support that spaces the LEDs from the user and further distances the LEDs from the user with a top cover that is distanced from the user.

U.S. Publication Number 2009/0222070 discloses a "Capsule with Whole Body LED Photo-Therapy System".

Further, document US 2014 0202715 discloses a tanning LED bed wherein LED modules are regulated by current limiting circuits.

Known LED beds appear to be based on retrofitted tanning beds. This design raises several issues which include:
1). Tanning beds having a clamshell design and a top portion which may sit too far away from the body for light emitted from LEDs to effectively penetrate the body of a user and deliver therapeutic energy. The bottom portion of a retrofitted tanning bed may have the same problem with proximity because the LEDs may sit too far away from the body for optimal efficacy.
2). An acrylic cover of a tanning bed may distort the preset angle being emitted by the LEDs and may also reduce power output.
3). With a LED bed that is retrofitted from a tanning bed, it may be impracticable or impossible to simultaneously raise the temperature of all parts of the human body because the torso would heat up and get hotter than the legs or the arms, and in some instances even the legs would heat up faster and get hotter than the arms.
4). With a separation of several inches, i.e. typically more than about fifty millimetres, between the LEDs and the user, the temperature needed to elevate the skin to obtain skin dilation and further provide the best penetration of phototherapy may be difficult, impractical or even impossible to obtain. In such an application, the benefits provided by the LEDs may be reduced by the support and the distance between the user and the LEDs

### SUMMARY OF THE INVENTION

There is provided a LED therapy bed comprising:
a plurality of separately controlled LED modules, each module having a plurality of LEDs regulated by a current limiting circuit;
the plurality of LEDs being overdriven to increase light output beyond normal operating intensity and to further produce thermal heat from said plurality of LEDs;
each module further including at least one thermal sensor that locally senses a temperature of said module; and
each module further including at least one fan wherein the fan speed is regulated directly or indirectly by the at least one thermal sensor; and
a master controller that controls each LED module, the at least one thermal sensor and the at least one fan;
wherein the temperature of each module or LED is separately controlled.

The LED therapy bed may comprise a support frame having a plurality of rails forming a curved support.

The plurality of rails may support the plurality of separately controlled LED modules.

Exemplary embodiments provide a custom designed bottom portion that has the LEDs virtually right up against an acrylic or other material cover, such that the LEDs are only about 6.4-12.7 mm (1/4-1/2 inch) from the users body, where a user would treat one side of their body and then flip over and treat the other side of their body. Alternatively, both sides of a user's body can be treated at the same time by having another bed positioned above the user that can be lowered into contact or near contact with the user. This arrangement solves the problem of the LEDs being spaced too far from the patient.

The modules of LEDs may be controlled individually or in groups, so that one part of the user will not be at too high or too low a temperature, compared to other parts of the user's body. In order to gain FDA clearance, it is necessary to simultaneously raise the temperature of all parts of the human body, e.g., the torso, the legs, the arms, neck, etc. Additionally, these modules are able to maintain the temperature regardless of normal variations in environmental or other conditions, like ambient temperature. Such an arrangement solves the problem of having a different temperature of different parts of a user's body, where, for example, a user's arms or legs may be at a different temperature than the user's torso, and the problem of different environmental or other conditions causing different body temperatures.

The thermal heat from said plurality of LEDs may be arranged to produce a skin temperature of a user of between 36°C and 42°C (97°F and 108°F).

The LED therapy bed may comprise an acrylic polymer cover over the LEDs, preferably the acrylic polymer is a transparent acrylic polymer. The acrylic polymer does not distort or minimize distortion of the angle of the LED energy being emitted, and lets almost all of the energy pass through to the user for therapeutic effect. This solves the problem associated with distortion of the angle of LED energy being emitted. Transparent or ultra-transparent acrylic polymer may be used.

The LEDs of an LED module may be arranged in an array. The LED modules may also be arranged in an array.

The LEDs of an LED module may be contained on a printed circuit board (PCB).

Each of the separately controlled LED modules may have electrical connections for between 100 to 1,000 LEDs in each module.

The plurality of LEDs, for example an array of LEDs may have different wavelengths which emit different colored light and are placed in a repeating pattern, for example an array. Alternatively, three-colour rgb LED may be controlled to produce desired wavelengths across individual or multiple LEDs within an array.

The LEDs may transmit light in the wavelengths of at least one of 605nm, 630nm, 660nm, 850nm to 855nm and 940nm. These wavelengths alone or in combination may cause at least one of: increase the nitric oxide level of a user of the LED therapy bed, reduce or eliminate wrinkles and tighten a user's skin, provide muscle or joint or arthritic or carpal-tunnel pain and/or stiffness relief, blood circulation, psoriasis, eczema, postop to reduce scarring, bruising, healing time, pain, inflammation and redness, healing sores in the mouth caused by chemo-therapy, to reverse blindness caused by diabetes, to reverse macular degeneration, to reverse loss of sight caused by stroke, to treat skin cancer, to reduce or eliminate bruises, to clear sinuses, to regrow hair and to treat Fibromyalgia.

The current limiting circuit may be configured to increase heating of at least one wavelength of LEDs to heat said skin tissue temperature to between 36°C and 42°C (97°F and 108°F) when held continuously against, or in close proximity to, the skin tissue for a predetermined period of time.

The thermal sensor may be located between LEDs on a spring arm which is spring biased.

The master controller controls the voltage delivered (and thus the current produced within each LED given its internal resistance) to LEDs in each array within each LED module, or otherwise commonly controls an entire array of LEDs in one module.

The master controller may separately control each LED module. Alternatively, the master controller may separately or individually controls some of the LED modules and controls other LED modules together, or as a group.

The plurality of LEDs may be placed in a repeating pattern, for example in an array.

The LED therapy bed may further comprise: an acrylic polymer cover over the LEDs; wherein the plurality of LEDs have the same wavelength or different wavelengths; wherein the master controller controls the LEDs.

The current limiting circuit may be selected to cause a deliberate increase in thermal heating of at least one wavelength of LEDs to heat said skin tissue temperature to between 36°C and 42°C (97°F and 108°F) when held continuously against, or in close proximity to, the skin tissue for a predetermined period of time.

The thermal sensor may sit between the LEDs on a spring arm, preferably the spring arm is spring biased. Whilst thermal sensors may be located proximate to, or on a surface of, the LEDs closest to the acrylic polymer cover, other forms of detector may substitute or augment the temperature sensing function performed by the thermal sensors. For example, pressure sensors may underlie or be incorporated within or on the acrylic polymer cover, with feedback of localised pressure readings providing a further mechanism by which the controller can control energy delivery to individual or groups of the LEDs. For example, depending upon the detected pressure, the LEDs can be overdriven to a higher or lower extent; this saves energy when no pressure reveals no contact. Moreover, by building a reported pressure profile based on weight distribution across the entirety of the surface of the therapy bed, LED energy output may be varied accordingly by the master controller. This arrangement can furthermore avoid areas of the bed that are relatively hot where wicking away of heat does not occur due to lack of body contact at a supporting surface of the acrylic polymer cover.

The acrylic polymer cover or layer may have a varying thickness across its surface, with the varying thicknesses presenting a form of thermal power attenuation in localised regions; this allows for groupings of LEDs to be commonly overdriven, with the thermal properties of the acrylic polymer cover moderating localised heating. Such an arrangement may eliminate the need for the master controller by reducing the controller to an on-off switch function. LED and thus thermal control may be achieved by pulse width modulation "PWM" of a controlling waveform applied to a drive voltage. The generation of a representative signal reflecting localized temperature can provide a feedback loop that controls timing of actuation, or level of applied overdriving voltage/current, of each LED or group of LEDs.

Heating effects produced across the acrylic polymer cover may therefore be substantially homogenous across all modules, or otherwise variations in localized heating may be practiced to address different qualities of skin on different parts of a body (or region of a body).

The LED therapy bed comprising a plurality of separately controlled LED modules or a plurality of separately controlled LED modules alone may comprise a system for light therapy which utilizes non-coherent light generated by an array of conventional light emitting diodes (LEDs) which are confined within a bandwidth of 415nm to 940nm. The diode array is configured in a matrix to direct the light onto a diffused area of the user without utilizing an optical system or any intermediary material other than the acrylic polymer layer. The LEDs rest directly on, or in close proximity to, the user through the use of the acrylic polymer layer or similar layer.

The LED therapy bed comprising a plurality of separately controlled LED modules or a plurality of separately controlled LED modules alone may overdrive the LEDs to create heat that is conducted to the skin of the user to provide heat in addition to the light therapy. In some embodiments a single current regulating, limiting device or a single resistor is used to consistently limit the current to all of the LEDs and provide both even illumination and heat.

The LED therapy bed comprising a plurality of separately controlled LED modules or a plurality of separately controlled LED modules alone may provide a light therapy system of the general character described, which produces beneficial bio-stimulative effects.

The LED therapy bed comprising a plurality of separately controlled LED modules or a plurality of separately controlled LED modules alone may provide a light therapy method of the general character described whereby non-coherent and non-monochromatic light within a wavelength range of 415nm to 940nm is employed for photo-bio-stimulation.

The LED therapy bed comprising a plurality of separately controlled LED modules or a plurality of separately controlled LED modules alone may provide a light therapy method of the general character described which utilizes non-coherent and non-monochromatic light emanating from the LEDs.

The LED therapy bed comprising a plurality of separately controlled LED modules or a plurality of separately controlled LED modules alone may include multiple modules of LED banks where each bank includes an individual thermal control of the module to maintain optimal skin dilation temperature. Each module may include 608 LEDs arranged in 19 rows by 32 columns. More or less columns and/or rows may be provided, including patterns other than columns and/or rows, such as circular shape, chevron shape, diagonal shape, etc.

The LED therapy bed comprising a plurality of separately controlled LED modules or a plurality of separately controlled LED modules alone may have an open top surface that does not cover over a person. The bed only includes LEDs under the user where the distance between the LEDs and the user can be maintained. Each bed may have banks of modules arranged in three rows by six columns.

In a further embodiment, one or more fans for cooling individual modules in order to regulate the temperature transmitted to the acrylic cover may be used. These cooling fans may drive a circulating air current within a plenum below the array of LEDs, the plenum forming part of the structural compartment that is each module or one or more sections of the therapy bed, with each section containing one or more modules. Again, rather than regulating a drive current to each LED or each array, a temperature sensor within each plenum and located proximate to each array (and preferably proximate to a heat transferring surface of the LEDs of the array) may be used to provide an indication of temperature at a thermal transfer interface. In response to the sensed temperature, the master controller may regulate the speed of the fan to regulate a temperature generated at the surface of the LED array, with the master controller arranged to execute a control algorithm that varies the temperature profile with time. Of course, embodiments consider that a delivered temperature at the thermal interface between a body under therapy and the acrylic polymer cover (or layer) may be substantially constant for a selected period of time ranging, for example, between tens of seconds to several minutes.

In addition or as an alternative, cooling fans may be positioned at on the surface to the acrylic polymer cover to blow regulating air over the surface, with surface mount temperature sensors providing feedback for fan control and/or power control of the LEDs.

The modules may, furthermore, include a surface that is perforated such that cooling air circulating with the plenum from the fan and egress through perforations to produce localized cooling of skin in contact with the acrylic polymer surface. Fan operation in this embodiment may be continuous or timed for selected periods controlled by the master control (to reflect a program of heat and light delivery from the entire therapy bed or isolated and selected parts of each of the modules or arrays.

The benefits of the LEDs may be logarithmically proportional to the distance between the LED and the user.

Various objects, features, aspects and advantages of the present invention will become more apparent from the following detailed description of preferred embodiments of the invention, along with the accompanying drawings in which like numerals represent like components.

The LED therapy bed may comprise a support frame having a plurality of rails forming a curved support.

The plurality of rails may support the plurality of separately controlled LED modules.

The thermal heat from said plurality of LEDs may be arranged to produce a skin temperature of a user of between 36°C and 42°C (97°F and 108°F).

The LED therapy bed may support the body of a user lying on the LED therapy bed but does not cover the user while the user is lying on the LED therapy bed.

After one side of a user has been treated by the modules, the user may turn over to have the opposite side of the user's body treated.

At least one of the following may be caused through use of the LED therapy bed: increase in the nitric oxide level of a user; reduction or elimination of wrinkles and tightening of a user's skin; providing muscle or joint or arthritic or carpal-tunnel pain and/or stiffness relief, blood circulation, psoriasis, eczema; post-op to reduce scarring, bruising, healing time, pain, inflammation and redness; healing sores in the mouth caused by chemo-therapy; to reverse blindness caused by diabetes; to reverse macular degeneration; to reverse loss of sight caused by stroke; to treat skin cancer; to reduce or eliminate bruises; to clear sinuses; to regrow hair and to treat Fibromyalgia.

The therapy bed comprises a support frame having a plurality of rails; the plurality of rails forming a curved support; the plurality of rails supporting a plurality of separately controlled LED modules.

In an embodiment, a LED therapy bed includes a support frame having a plurality of rails. The plurality of rails form a curved support. The rails support a plurality of separately controlled LED modules, where each module has a plurality of LEDs regulated by a current limiting circuit. Said plurality of LEDs being overdriven to increase light output beyond normal operating intensity and to further produce thermal heat from said plurality of LEDs in order to produce a skin temperate of a user of between 36°C and 42°C (97°F and 108°F) from direct thermal conduction, the temperature measured at or near a surface of the acrylic polymer. Each module further including at least one thermal sensor that locally senses a temperature of said module; and each module further including at least one fan wherein said fan speed is regulated directly or indirectly by said at least one temperature sensor. A master controller that controls each said module LEDs, the thermal sensor and the at least one fan; wherein the temperature of each module can be separately controlled.

In a further embodiment, a LED therapy bed includes a support frame having a plurality of rails forming a curved support. The rails support a plurality of LED modules. Each module having a plurality of LEDs regulated by one or more current limiting circuits. The plurality of different colored LEDs being overdriven to increase light output beyond normal operating intensity and to further produce thermal heat from said plurality of LEDs in order to produce a skin temperate of a user of between 36°C and 42°C (97°F and 108°F) from direct thermal conduction. Each LED module further including at least one thermal sensor that locally senses a temperature of said module. Examples of known acrylic are transparent or ultra-transparent acrylic. However, other acrylics may be used as well. The cover is located over the top of the LEDs and space the LEDs from the user. Each module further including at least one fan wherein said fan speed is regulated directly or indirectly by said at least one temperature sensor. A master controller controls the LEDs, the at least one fan and the at least one thermal sensor in each module; wherein the temperature of each module can be separately controlled.

Advantageously, embodiments provide a phototherapy device that provides regulated skin temperature so that all areas of the body can be brought up to a therapeutic temperature level simultaneously and held at that temperature. Furthermore, the structural arrangement of the devices of at least some of the preferred embodiments achieve localized, regulated hearing regardless of non-optimum environmental conditions, like ambient temperature, whilst further providing very close dermal phototherapy to the light sources.

### BRIEF DESCRIPTION OF THE DRAWINGS

FIG. 1 shows a perspective view of a LED therapy bed;
FIG. 2 shows a perspective cross-sectional view of the LED therapy bed, taken along lines 2-2 of FIG. 1;
FIG. 3 shows a perspective cross-sectional view of the LED therapy bed, taken along 3-3 of FIG. 1;
FIG. 4 shows a perspective view of a single LED module;
FIG. 5 shows a perspective sectional view of the single LED module, as taken along line 5-5 of FIG. 4;
FIG. 6 shows a detailed view of the LED module, according to detail 6, identified in FIG. 2;
FIG. 7 shows a detailed view of the LED module, according to detail 7, identified in FIG. 3;
FIG. 8 shows a block diagram of the LED therapy bed according to the invention;
FIG. 9 shows the thermistor thermal sensor of FIG. 9 in greater detail; and
FIG. 10 shows the thermistor thermal sensor of FIG. 9 placed in an LED module.

### DETAILED DESCRIPTION OF THE INVENTION

For the purposes of promoting an understanding of the principles of the invention, reference will now be made to exemplary embodiments illustrated in the drawings and specific language will be used to describe the same. It will nevertheless be understood that no limitation of the scope of the invention is hereby intended. Any alterations and further modifications of the principles of the invention as described herein are contemplated and would normally occur to one of ordinary skill in the art to which the invention relates. The invention is defined in the claims; other embodiments, aspects and methods are provided for illustrative purposes only and are not parts of the invention.

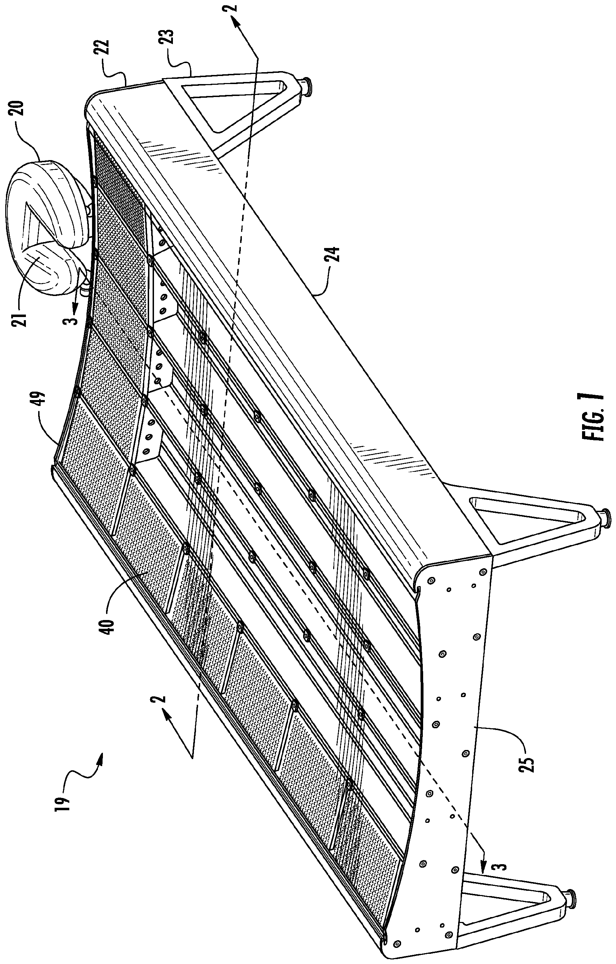

FIG. 1 shows a perspective view of an LED therapy bed 19, according to an exemplary embodiment. The LED therapy bed 19 is configured as having an open top structure. A plurality of legs 23 suspends a frame structure above the ground. The frame structure has front and rear frame members 22 and 25 with elongated side members 24 there between. As shown in FIG. 1, the bed has a plurality of modules 40, as discussed below in further detail. At one or both ends of the therapy bed 19 is a head rest/face rest 20 which has inner side supports 21. Element 20 serves as a headrest when a user is laying on their back on the LED therapy bed, and serves as a face rest when a user is lying face down on the LED therapy bed 19. A space between the inner side supports 21 serves to receive the face of a user who is lying face down on the LED therapy bed 19. As additionally shown in FIG. 1, an acrylic cover 49 rests on the LED therapy bed 19 and is between the user and the LED modules 40. The acrylic cover or layer 49 is designed to lower the temperature between the bottom of the cover, which rests on the LEDs and the frame of the modules and the temperature of the top of the cover, which receives the body of the user. The temperature of the top of the acrylic cover 49 which receives the body of the user is at a temperature of between 36°C and 42°C (97°F and 108°F), depending on the therapy to be provided. Although an acrylic cover is disclosed, other suitable materials may be used for a transparent or nearly transparent acrylic cover that rests on the LEDs in the LED modules. In the exemplary embodiment illustrated, the weight of the user presses down on the acrylic layer 49 such that the LEDs are in contact with the body through the acrylic layer.

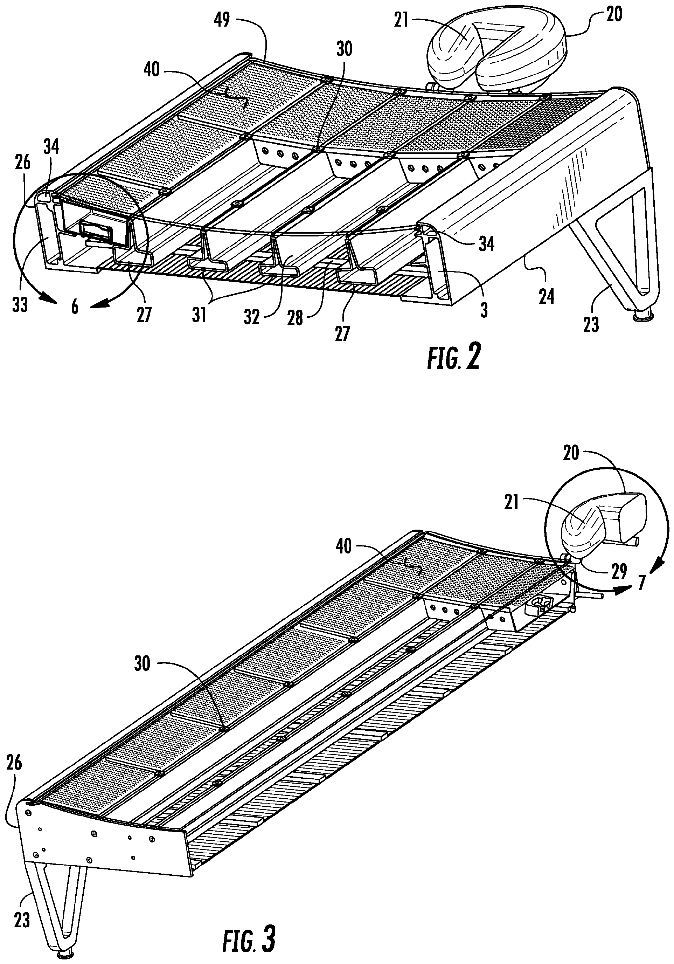

FIG. 2 shows a perspective view taken along lines 2-2 in FIG. 1. Lengthwise extending frame members extend from one end of the therapy bed 19 to an opposite end. The frame members have an upper portion which is in an inverted "V" shape. Connected to the bottom of each inverted frame member is a horizontally extending member 31. Two horizontally extending members in the center portion are of the same height. The two elongated frame members on either side of the two centrally located horizontally extending members are labeled as 27 and have a larger height than the centrally located horizontally extending members, which are labeled as 31. Thus, both horizontally extending members 31 are of the same height and both elongated frame members 27 are of the same height. Outside of the horizontally extending members are outer members which are hollow and are labeled as 33. Above the outer tubular members 33 are hollow upper portions 34. Extending crosswise between the frame members are frame supports 28, which extend crosswise under the center of each LED module 40. Although a specific frame structure has been described, other frame structures may be substituted. One or both ends of the LED therapy bed 19 has a head or face rest 20 with inner side supports 21 which support a person that is lying on the LED therapy bed 19 on their back or on their face, in order to enable the person using the LED therapy bed 19 to receive therapeutic benefits to either front, back or sides of the user. Sides of the user are treated by a user laying on one side and then, if needed, on their other side. A plurality of removable and replaceable LED modules 40 are placed in the frame of the LED therapy bed 19. The transparent acrylic cover 49 provides a slight spacing between the user and the LEDs. The transparent acrylic cover 49 distributes the weight of the user on the frame structure of the plurality of removable and replaceable LED modules 40. The transparent acrylic cover 49 is preferably made from a clear material, such as acrylic or polycarbonate, but other materials may be used that provide equivalent or superior transparency or structural strength.

As can be additionally seen in FIG. 2, elongated side members 24 and 26 are above legs 23. Between the elongated side members 24 and 26, elongated frame members 27 and 31 support the LED modules 40 in a curved configuration that centers the user in the center of the LED therapy bed 19. The head rest or face rest 20 supports the rear of the head of a user and can support the face of a user and provides clearance for the user to breathe through their nose or mouth. A support 29 connects an end of the LED therapy bed 19 and the head rest or face rest 20. Although one head rest or face rest 20 has been shown, the head rest or face rest 20 can be located on either end of the therapy bed. In one exemplary embodiment, the LED therapy bed 19 has thirty LED modules 40 configured in five columns and six rows, but LED therapy beds with more or less modules and a different numbers of columns and rows of modules may be provided. Each LED module 40 is essentially the same, but may be of different shape or size, and each LED module 40 can be removed, replaced or swapped. Each LED module 40 is self-contained and can operate independently or together with any or all other LED modules 40. The LED modules 40 are shown and described in more detail in other figures herein.

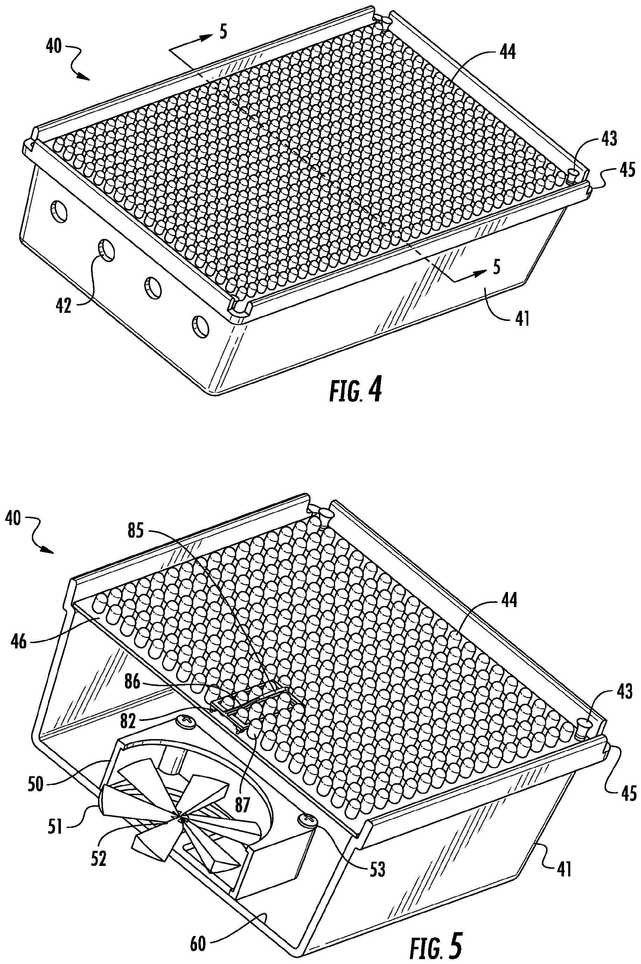

FIG. 3 shows a perspective view of FIG. 1, taken along line 3-3. A rectangular connector 30 is provided. This connector 30 fits in the cutaway portions 45 (see FIG. 4) at the corners of each LED module 40. The corners of four modules form a rectangle which receives rectangular connector 30 to hold the LED modules 40 in place. The top of the connector 30 is below the top of the LEDs, in an exemplary embodiment.

FIG. 4 shows a perspective view of a single LED module 40. In one exemplary embodiment, each LED module 40 has a matrix of LEDs configured in 19 by 32 columns for a total of 608 LEDs, but other embodiments of rows and columns of LEDs may be provided, instead of the particular configuration shown. The LEDs provide non-coherent light generated by an array of conventional light emitting diodes (LEDs) which are confined within a bandwidth of about 415nm to about 940nm. The LED array is configured in a matrix to direct the light onto a diffuse area of the user, through the acrylic layer, without utilizing an optical system, etc. The light is emitted at a preset angle to provide the most effective treatment of a user. Housing 41 supports the internal electronics and a circuit board 46 (see FIG. 5) which supports the LEDs 44. Each individual LED 44 module 40 may be secured to the housing 41 with fasteners 43. In addition, in one exemplary embodiment, one or more of the separately controlled LED modules 40 may have electrical connections for between about 100 to about 1,000 LEDs 44.

Each LED module 40 is self-contained and independently regulates its temperature to maintain an optimal skin dilatation temperature. A current limiting device connects to the LEDs. In an exemplary embodiment, the current limiting resistor is selected to provide a deliberate increase in said skin tissue temperature of a user where the skin temperature is between 36°C and 42°C (97°F and 108°F), when held continuously against or in very close proximity to a user's skin tissue for a fifteen-minute period. Each LED module 40 has openings or holes 42 for venting air from the inside of the LED module 40. The holes 42 allow for cooling or heating air to be independently moved through each LED module 40 to independently regulate the temperature of each LED module 40. As shown in FIG. 4, the top portion of LED module 40 overhangs the housing 41 so that the air from the holes 42 is vented away from each LED module 40. In addition, each module has one or more fans 51 to move air within the module, in order to control the temperature of the air below acrylic cover 49, which results in control of the temperature of the top of the acrylic cover, which is in contact with the body of the user. Individual control of the temperature of the air above and below the acrylic cover is important because different portions of a user's body put out more heat than other portions. For example, a user's legs put out more heat than the user's trunk; whereas the user's arms may put out more heat than the user's legs, etc. As an alternative, the LED therapy bed 19 may be formed from two modules. In addition, a second set of modules may be lowered onto or otherwise placed on the other side of the user to treat both sides of the user at the same time. Moreover, the bed or upper and lower beds may be vertically oriented or oriented at an angle to the vertical or horizontal.

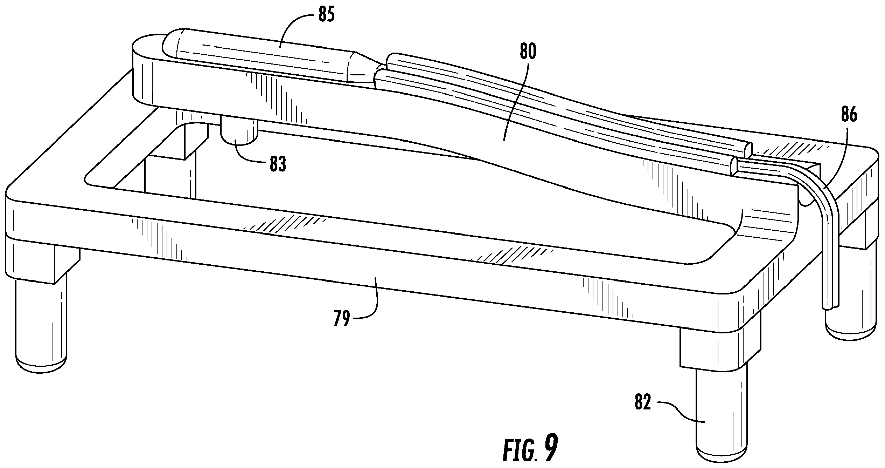

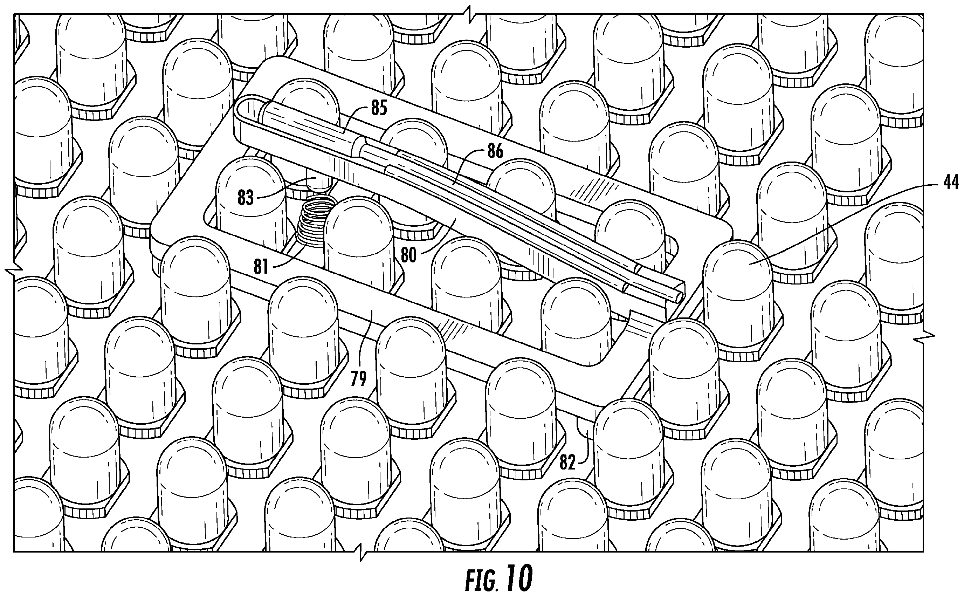

FIG. 5 shows a perspective sectional view of the single LED module 40 from FIG. 4, taken along line 5-5 in FIG. 4. Each module 40 can have a heating element (not shown) that can preheat each module independently in order to reduce the time required to obtain the desired therapy temperature for a particular user. A thermistor, other heat sensor or temperature sensor 85 is located at the top of each LED module 40 to determine the temperature of each LED module 40. The thermistor, other heat sensor or temperature sensor 85 sits on a frame and is supported on legs 82. An illustration of the thermistor or other heat or temperature sensor can be found in FIGS. 9 and 10.

A controller is located either within each LED module 40 or at a separate master location. The controller measures the temperatures through sensors 85 and operates the fan 51 that is connected to a motor 52 found in compartment 50 at the bottom of the LED module 40. The fan 51 has blades and the fan speed changes to maintain the surface temperature of the LEDs 44 at the underside of acrylic cover 49. The fan compartment 50 is kept in place by screws 53, or is otherwise secured to the housing 41 of LED module 40. The regulation of the fan speed and cooling is required because the LEDs 44 are overdriven to create heat that is conducted to the skin of the user to provide heat in addition to the light therapy.

In some exemplary embodiments, the power applied to the bank of LEDs is through an LED driver. The LED driver can be in a variety of forms, from a simple resistor to a transistor, SCR, current driver, Diac, Triac or other solid state device. The power to a module of LEDs or to each LED 44 is supplied at a desired power or current, as controlled by the controller. The current regulating or limiting device is used to consistently limit the current to the LEDs and provide both even illumination and the specific temperatures needed. Because the LEDs are often driven beyond their normal level of illumination, the LEDs produce excessive heat. Excessive heat is exhausted from each LED module 40 through holes 42, as a result of the air flow created by fan 51.

Different wavelengths of light and combination of wavelengths of light have been shown to provide various treatments including but not limited to:
1. Treatment of wrinkles/anti-aging, and to reduce pore size: about 605nm, about 630nm, about 660nm, and about 850nm to about 855nm.
2. Pain relief including carpal-tunnel and arthritic pain: about 630nm, about 660nm, about 850nm to about 855nm, and about 940nm.
3. Treat acne and healing burn victims: about 415nm or about 460nm to about 465nm, about 660nm, and about 850nm to about 855nm.
4. Rosacea: about 415nm or about 460nm to about 465nm, about 630nm, about 660nm, and about 850nm to about 855nm.
5. MRSA: about 415nm or about 460nm to about 465nm, and about 850nm to about 855nm.
6. Treat swelling and inflammation of the brain caused by severe head trauma: about 850nm to about855nm.
7. Psoriasis + Eczema (used w/serum): about 630nm, about 660nm, about 850nm to about 855nm, and about 940nm.
8. Post-op to reduce scarring, bruising, healing time, pain, inflammation and redness: about 630nm, about 660nm, about 850nm to about 855nm, and about 940nm.
9. Reverse blindness caused by diabetes: about 630nm, about 660nm, about 850nm to about 855nm, and about 940nm.
10. Reverse macular degeneration: about 630nm, about 660nm, about 850nm to about 855nm, and about 940nm.
11. Heal sores in the mouth caused by chemo-therapy: about 630nm, about 660nm, about 850nm to about 855nm, and about 940nm.
12. Skin cancer: about 630nm, about 660nm, about 850nm to about 855nm, and about 940nm.
13. Bruising: about 630nm, about 660nm, about 850nm to about 855nm, and about 940nm.
14. Sinuses: about 630nm, about 660nm, about 850nm to about 855nm, and about 940nm.
15. Bell's Palsy: about 630nm, about 660nm, about 850nm to about 855nm, about 940nm, about 605nm, about 630nm, about 660nm, and about 850nm to about 855nm.
16. Heal the chest after open-heart surgery: about 850nm to about 855nm.
17. Help to re-grow hair: about 630nm, about 660nm, about 850nm to about 855nm, and about 940nm.
18. Fibromyalgia: about 630nm, about 660nm, about 850nm to about 855nm, about 940nm, about 605nm, about 630nm, about 660nm, and about 850nm to about 855nm.
19. Increase of Nitric Oxide production: about 630nm, about 660nm, about 850nm to about 855nm, and about 940nm.
20. Increased blood circulation: about 630nm, about 660nm, about 850nm to about 855nm, and about 940nm.
21. Pigmentation and age spots: about 605nm, about 630nm, about 660nm, and about 850nm to about 855nm.

The plurality of light can have a small variation between the light frequencies, such as about 625nm, about 630nm and about 635nm, by using LEDs with different dispersion and intensities. These light frequencies, about 625nm, about 630nm and about 635nm can be combined with a light frequency of about 415nm that kill bacteria to provide optimal benefit. Although specific wavelengths are described above, the wavelengths can be modified, if desired. In addition, although the term "about" is used in the specification when listing specific wavelengths, the term "about" is used because manufacturing tolerances may differ and because a very similar but not exact wavelength may work as well as the listed wavelength.

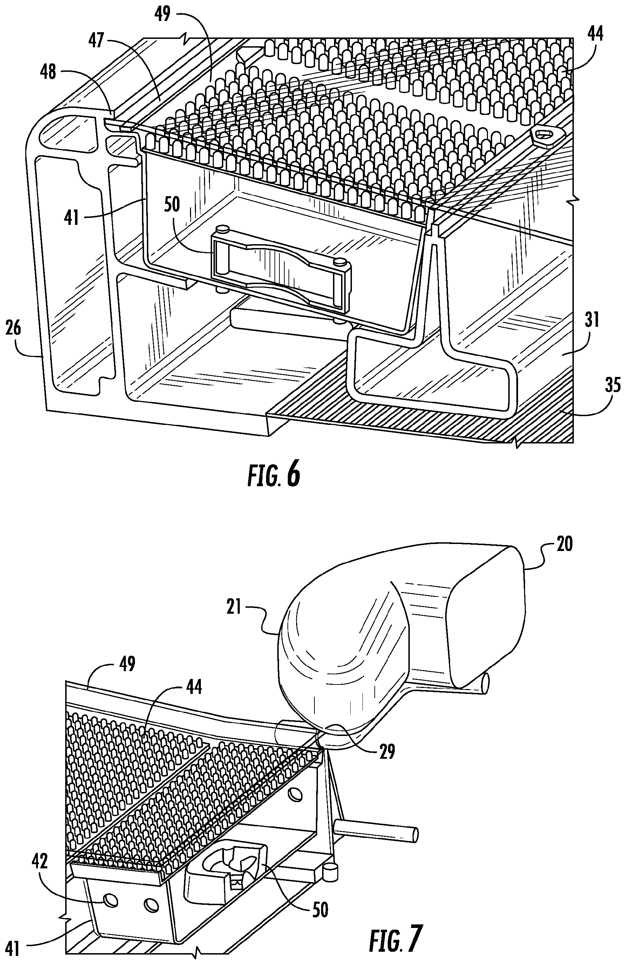

FIG. 6 shows a detailed view of the LED module 40 from the detail 6 identified in FIG. 2. The LED module 40 is shown installed and retained in the top of side frame member 26. At the top of side frame member 26 is a hollow upper portion 34 (see FIG. 2). As further shown in FIG. 6, a side of the acrylic cover 49 rests on a rubber strip 47 and is kept in place from moving upwardly by overhang 48 of the hollow upper portion 34 of side frame member 26. These sections show the fan compartment 50 that provides air flow to maintain the temperature of the LED module 40, and in particular, to control the air temperature at the bottom and top of the LEDs which are beneath acrylic cover 49. Below rails 28 is a mesh fabric 35 which is made of metal or another suitable material, and which extends across most of the underside of the LED therapy bed.

FIG. 7 shows a detailed view of the LED module 40 from the detail 7 identified in FIG. 3. The head rest or face rest 20 supports the rear of the head of a user and can support the face of a user and provides clearance for the user to breathe through their nose or mouth. A support 29 is connected between frame member 26 of the LED therapy bed 19 and the head rest or face rest 20. Although a frame is shown supporting the modules, other different frames may be substituted. In addition, although a plurality of modules 40 are shown to be located below the user, the modules 40 can also be placed above the user. In addition, sets of modules 40 can be place both above and below the user, as well as to cover the sides of the user. Moreover, although the modules 40 are described as being essential horizontal, they can alternatively be placed in a vertical orientation or at an angle from the vertical or horizontal axes.

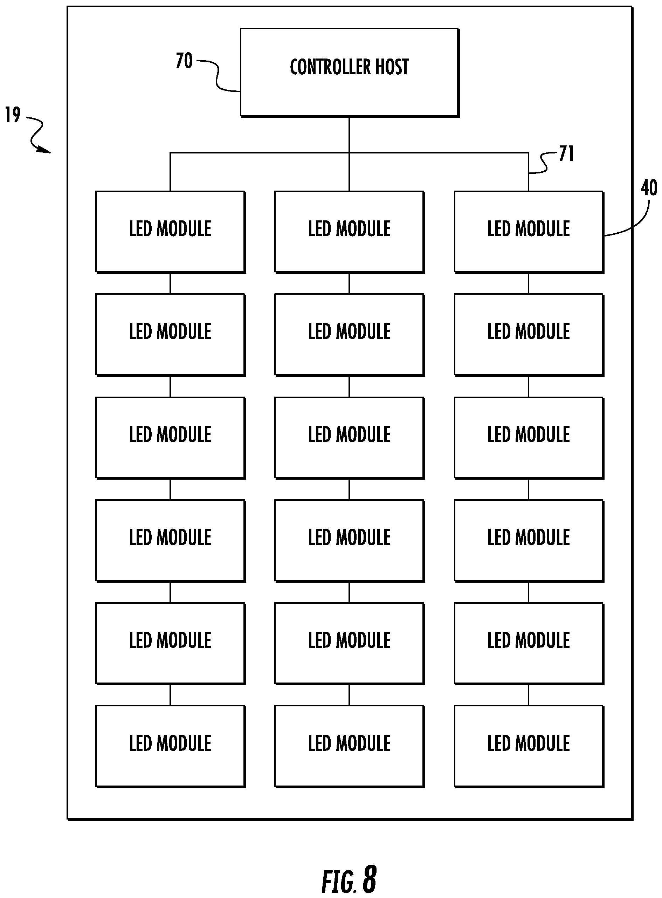

FIG. 8 shows a block diagram of the LED therapy bed 19. A controller 70 is wired at 71 to each of the LED modules 40. The connection from the controller 70 to the LED modules 40 can be a direct connection to each LED module 40 or can be connected in a serial or daisy chain method. The controller 70 is the master controller and each LED module 40 is a slave unit to controller 70. The host operates the display and a keyboard or knobs that accept user input, and operates the display, indicators, sound making devices etc., and the slave unit(s). Each slave LED module 40 has their own controller that controls the LEDs, fan, and monitors the temperature sensors.

FIGs. 9 and 10 show the temperature sensor 85 is located at the end of a flexible arm 80 that rests on or is secured on the LED matrix circuit board via a support structure. The flexible arm 80 retains the temperature sensor 85, which may be a thermistor, in conductive contact or nearly conductive contact with the underside of the transparent acrylic cover 49 and accommodates some flexing of the transparent acrylic cover 49 that is supported on the frame containing the LEDs 44. It is also contemplated that a thermal image temperature sensor can be used that does not rely upon conductive contact with the underside of the transparent acrylic cover 49. On the underside of the flexible arm 80 is a projection 83. This projection 83 may press downwardly onto the top of spring 81 when the arm is downwardly flexed. The thermistor 85 or equivalent rests in a groove in the top surface of the flexible arm 80. A wire or wires 86 extend from thermistor 85 across the flexible arm 80 and down to the printed circuit board (PCB) that the LEDs 44 are supported on. The flexible arm 80 is supported on a frame 79 which rests on legs 82. The frame is located between the LEDs 44 and the top of thermistor 85 is just below the top of the LEDs 44. While one temperature sensor is shown in this exemplary embodiment, multiple temperature sensors can be placed in the LED array to the underside of the transparent acrylic cover 49. Multiple temperature sensors 85 allow for reduction in the conduction of heat/cooling from a part of the user's body placed on the transparent acrylic cover 49. In addition to temperature sensors placed between the LEDs and below the transparent acrylic cover 49, additional temperature sensors can be located on the (PCB) and/or elsewhere within the LED module. The temperature sensors 85 communicate with the module where a controller 70 in each module, or a central controller, which operates one or more cooling fans 51 to maintain the temperature of the module 40.

At least one temperature sensor 85 is held in near contact with the bottom surface of the transparent acrylic cover 49 by being located just below the top of the LEDs 44. As previously described, each LED module 40 has six-hundred and eight LEDs but more or less than the six-hundred and eight LEDs may be provided. The number of wavelengths of the LEDs utilized is selected based upon the desired therapy. In addition, the LEDs can be fabricated with an internal cluster of LEDs and the transmission color of each individual LED 44 can be changed, based upon the desired therapy. In an exemplary embodiment, a plurality of different colored LEDs may be placed in a repeating pattern. Skin and other body tissues have the ability to absorb light and use it as a source of energy to stimulate cellular regeneration. The light rays that are emitted from the device are beneficial for your skin, as they contain no UV rays. The problem with getting these same light rays from the sun is that you also get the harmful UV rays. These harmful rays can do more damage to your skin than good. With LEDs, when the correct wavelengths of light are closely and intensely flowed into the body at the proper temperatures, collagen and elastin are produced by cells called Fibroblasts. Inside these cells is a smaller cellular structure called Mitochondria.

## Claims

1. A LED therapy bed (19) comprising:
a plurality of separately controlled LED modules (40);
each module having a plurality of LEDs regulated by a current limiting circuit; the bed being **characterised in that** the plurality of LEDs is adapted to be overdriven to increase light output beyond normal operating intensity and to further produce thermal heat from said plurality of LEDs;
each module further including at least one thermal sensor (85) that is adapted to locally sense a temperature of said module; and
each module further including at least one fan (51) wherein the fan speed is regulated directly or indirectly by the at least one thermal sensor; and
a master controller that is adapted to control each LED module, the at least one thermal sensor and the at least one fan;
wherein the temperature of at least more than one of the modules or LEDs is separately controlled.

2. The LED therapy bed according to claim 1, further comprising a support frame having a plurality of rails; the plurality of rails forming a curved support; the plurality of rails supporting the plurality of separately controlled LED modules.

3. The LED therapy bed according to any preceding claim, further comprising an acrylic polymer covering the LEDs.

4. The LED therapy bed according to claim 3, wherein the acrylic is a transparent acrylic polymer.

5. The LED therapy bed according to any preceding claim, wherein LEDs of an LED module are contained on a printed circuit board (PCB).

6. The LED therapy bed according to any preceding claim, wherein each of the separately controlled LED modules has electrical connections for between about 100 to about 1, 000 LEDs in each module.

7. The LED therapy bed according to any preceding claim, wherein the plurality of LEDs have different operating wavelengths to emit different colored light and are placed in a repeating partem.

8. The LED therapy bed according to any preceding claim, wherein the LEDs transmit light in the wavelengths of at least one of: 605nm, 630nm, 660nm, 850 to 855nm and 940nm.

9. The LED therapy bed according to any preceding claim, wherein the thermal sensor is located between LEDs on a spring arm which is spring biased.

10. A LED therapy bed according to any preceding claim, wherein the master controller controls the LEDs in each LED module.

11. The LED therapy bed according to any preceding claim, wherein the master controller separately controls each LED module.

12. The LED therapy bed according to any of claims 1 to 11, wherein the master controller separately or individually controls some of the LED modules and controls other LED modules together or as a group.

## Patentansprüche

1. LED-Therapiebett (19), umfassend:
eine Vielzahl von getrennt gesteuerten LED-Modulen (40);
wobei jedes Modul eine Vielzahl von LEDs aufweist, welche von einem Strombegrenzungs-Schaltkreis geregelt werden;
wobei das Bett **dadurch gekennzeichnet ist, dass** die Vielzahl von LEDs eingerichtet ist, um übersteuert zu werden, um die Lichtleistung über die normale Betriebsintensität hinaus zu erhöhen und mit der Vielzahl von LEDs ferner thermische Wärme zu erzeugen;
wobei jedes Modul ferner mindestens einen Wärmesensor (85) enthält, der eingerichtet ist, um eine Temperatur des Moduls lokal zu erfassen; und
wobei jedes Modul ferner mindestens einen Lüfter (51) aufweist, wobei die Lüfterdrehzahl direkt oder indirekt von dem mindestens einen Wärmesensor geregelt ist; und
wobei eine Hauptsteuerung eingerichtet ist, um jedes LED-Modul, den mindestens einen Wärmesensor und den mindestens einen Lüfter zu steuern;
wobei die Temperatur von mindestens mehr als einem der Module oder LEDs separat gesteuert ist.

2. LED-Therapiebett nach Anspruch 1, ferner umfassend einen Stützrahmen mit einer Vielzahl von Schienen; wobei die Vielzahl von Schienen einen gekrümmten Träger bilden; wobei die Vielzahl von Schienen die Vielzahl von separat gesteuerten LED-Moduls trägt.

3. LED-Therapiebett nach einem der vorgehenden Ansprüche, ferner umfassend ein Acrylpolymer, welches die LEDs abdeckt.

4. LED-Therapiebett nach Anspruch 3, wobei das Acryl ein transparentes Acrylpolymer ist.

5. LED-Therapiebett nach einem der vorgehenden Ansprüche, wobei LEDs eines LED-Moduls auf einer Leiterplatte (PCB) enthalten sind.

6. LED-Therapiebett nach einem der vorgehenden Ansprüche, wobei jedes der separat gesteuerten LED-Module elektrische Anschlüsse für etwa 100 bis etwa 1'000 LEDs in jedem Modul aufweist.

7. LED-Therapiebett nach einem der vorgehenden Ansprüche, wobei die Vielzahl von LEDs unterschiedliche Betriebswellenlängen aufweisen, um verschiedenfarbiges Licht zu emittieren, und in einem sich wiederholenden Muster angeordnet sind.

8. LED-Therapiebett nach einem der vorgehenden Ansprüche, wobei die LEDs Licht in den Wellenlängen von mindestens einer aus: 605 nm, 630 nm, 660 nm, 850 bis 855 nm und 940 nm emittieren.

9. LED-Therapiebett nach einem der vorhergehenden Ansprüche, wobei sich der Wärmesensor zwischen LEDs auf einem Federarm, der federvorgespannt ist, angeordnet ist.

10. LED-Therapiebett nach einem der vorgehenden Ansprüche, wobei die Hauptsteuerung die LEDs in jedem LED-Modul steuert.

11. LED-Therapiebett nach einem der vorgehenden Ansprüche, wobei die Hauptsteuerung jedes LED-Modul separat steuert.

12. LED-Therapiebett nach einem der Ansprüche 1 bis 11, wobei die Hauptsteuerung einige der LED-Module separat oder einzeln steuert und andere LED-Module zusammen oder als Gruppe steuert.

## Revendications

1. Lit thérapeutique à DEL (19) comprenant :
une pluralité de modules DEL à commande séparée (40) ;
chaque module présentant une pluralité de DEL réglées par un circuit de limitation du courant ;
le lit étant **caractérisé en ce que** la pluralité de DEL est configurée pour être suralimentée afin d'augmenter la sortie lumineuse au-delà de l'intensité lumineuse normale et afin de produire en outre de la chaleur à partir de ladite pluralité de DEL ;
chaque module comportant en outre au moins un capteur thermique (85) qui est configuré pour capter une température locale dudit module ; et
chaque module comportant en outre au moins un ventilateur (11) selon lequel la vitesse du ventilateur est réglé directement ou indirectement par le au moins un capteur thermique ; et
un contrôleur maître qui est configuré pour commander chaque module DEL, le au moins un capteur thermique et le au moins un ventilateur ;
selon lequel la température d'au moins plus d'un des modules ou de DEL est commandée de manière séparée.

2. Lit thérapeutique à DEL selon la revendication 1, comprenant en outre :
un châssis de support présentant une pluralité de rails ;
la pluralité de rails formant un support courbe ;
la pluralité de rails supportant la pluralité de modules DEL commandés séparément.

3. Lit thérapeutique à DEL selon l'une quelconque des revendications précédentes, comprenant en outre un polymère acrylique recouvrant les DEL.

4. Lit thérapeutique à DEL selon la revendication 3, selon lequel l'acrylique est un polymère acrylique transparent.

5. Lit thérapeutique à DEL selon l'une quelconque des revendications précédentes, selon lequel les DEL d'un module à DEL sont contenues dans une carte à circuit imprimé (PCB).

6. Lit thérapeutique à DEL selon l'une quelconque des revendications précédentes, selon lequel chacun des modules DEL à commande séparée présente des connexions électriques pour entre environ 100 à environ 1000 DEL dans chaque module.

7. Lit thérapeutique à DEL selon l'une quelconque des revendications précédentes, selon lequel la pluralité de DEL présente des longueurs d'ondes opérationnelles différentes afin d'émettre de la lumière colorée différente et est placée selon un motif répété.

8. Lit thérapeutique à DEL selon l'une quelconque des revendications précédentes, selon lequel les DEL transmettent de la lumière dans des longueurs d'onde d'au moins une longueur d'onde parmi 605nm, 630nm, 660nm, 850 à 855nm, et 940nm.

9. Lit thérapeutique à DEL selon l'une quelconque des revendications précédentes, selon lequel le capteur thermique est situé entre les DEL sur un bras de ressort qui est biaisé par un ressort.

10. Lit thérapeutique à DEL selon l'une quelconque des revendications précédentes, selon lequel le contrôleur maître commande les DEL dans chaque module à DEL.

11. Lit thérapeutique à DEL selon l'une quelconque des revendications précédentes, selon lequel le contrôleur maître commande séparément chaque module à DEL.

12. Lit thérapeutique à DEL selon l'une quelconque des revendications 1 à 11, selon lequel le contrôleur maître commande séparément ou individuellement certains des modules DEL et commande d'autres modules DEL ensemble ou sous forme de groupe.
